(19) Europäisches Patentamt
European Patent Office
Office européen des brevets

(11) Veröffentlichungsnummer: **0 123 241**
**B1**

(12) # EUROPÄISCHE PATENTSCHRIFT

(45) Veröffentlichungstag der Patentschrift: **08.03.89**

(21) Anmeldenummer: **84104258.3**

(22) Anmeldetag: **14.04.84**

(51) Int. Cl.⁴: **C 07 J 1/00**, C 07 J 5/00, C 07 J 7/00, C 07 J 17/00, C 07 J 41/00, C 07 J 51/00

(54) **Verfahren zur Herstellung von Pregnan-Derivaten.**

(30) Priorität: **25.04.83 DE 3315324**

(43) Veröffentlichungstag der Anmeldung: **31.10.84 Patentblatt 84/44**

(45) Bekanntmachung des Hinweises auf die Patenterteilung: **08.03.89 Patentblatt 89/10**

(84) Benannte Vertragsstaaten: **AT BE CH DE FR GB IT LI LU NL SE**

(56) Entgegenhaltungen:
**EP-A-0 113 812**
**DE-B-2 625 306**
**GB-A-1 043 675**
**US-A-3 067 214**
**US-A-3 211 725**

**TETRAHEDRON, Band 22, Nr. 8, August 1966, Seiten 2829-2836, Pergamon Press, Oxford, GB; C. BURGESS et al.: "Modified steroid hormones-XLVI. Some 17alpha-ethynyl and 17alpha-vinyl derivatives"**
**Chem. Ber. 111, 1978, Seiten 3086-3393**
**Fieser/Fieser: Reagents for organic synthesis, ISBN 047125875 Seiten 1018-1019**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Hofmeister, Helmut, Dr., Weislingenstrasse 4, D-1000 Berlin 28 (DE)**
Erfinder: **Laurent, Henry, Dr., Glambecker Weg 21, D-1000 Berlin 28 (DE)**
Erfinder: **Annen, Klaus, Dr., Osthofstrasse 80, D-4400 Münster- Albachten (DE)**
Erfinder: **Wiechert, Rudolf, Prof., Petzower Strasse 8A, D-1000 Berlin 39 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 123 241 B1

LIBER, STOCKHOLM 1989

**Beschreibung**

Die Erfindung betrifft ein Verfahren zur Herstellung von Pregnan-Derivaten der allgemeinen Formel I

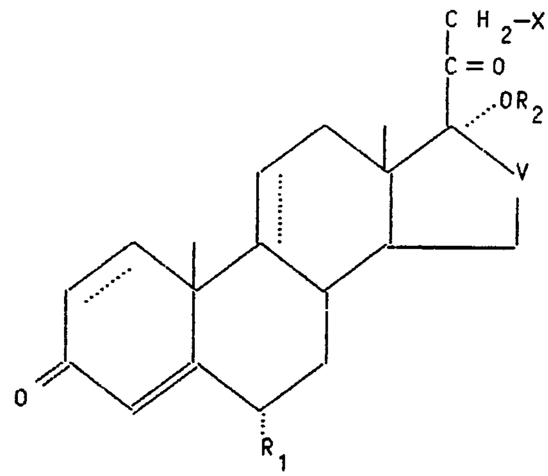

(I),

worin
.... Einfachbindungen oder Doppelbindungen darstellen,
$R_1$ ein Wasserstoffatom, ein Fluoratom oder eine Methylgruppe bedeutet,
$R_2$ ein Wasserstoffatom oder einen Formylrest symbolisiert,
X ein Bromatom oder ein Iodatom darstellt und
V eine Methylengruppe, eine Ethylengruppe, eine Ethylidengruppe oder eine Vinylidengruppe bedeutet, aus 17α-Ethinylsteroiden der allgemeinen Formel II

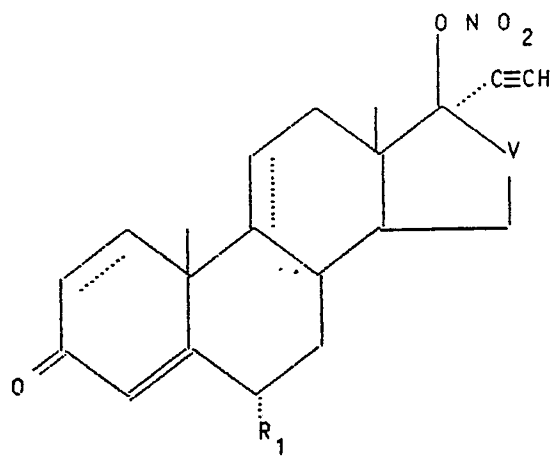

(II),

worin
...., $R_1$ und V die oben genannte Bedeutung besitzen.

Verfahren zur Herstellung von Pregnan-Derivaten der allgemeinen Formel I aus 17α-Ethinylsteroiden der allgemeinen Formel II sind vorbekannt.

So kann man beispielsweise aus den 17α-Ethinylsteroiden mittels des von Hofmeister et. al. beschriebenen Verfahrens (DE-A-2 625 306 und Chem. Ber. 111, 1978, 3086 - 3093) die entsprechenden in 21-Position unsubstituierten Pregnan-Derivate herstellen, welche dann ihrerseits nach dem von Stork beschriebenen Verfahren (J. Fried and J. E. Edwards "Organic Reactions in Steroid Chemistry" Vol II; van Nostrand Reinhold Comp. Hew York etc. 1972, p 207) in die 21-Jodverbindungen der allgemeinen Formel 1 überführt werden können. Diese vorbekannten Verfahren sind aber recht aufwendig und die hierbei erzielten Ausbeuten unbefriedigend.

Es wurde nun gefunden, daß man die Pregnan-Derivate der allgemeinen Formel I in einfacher Weise und unter Erzielung höherer Ausbeuten aus den 17α-Ethinyl-steroiden der allgemeinen Formel II darstellen kann, indem man diese in einem inerten Lösungsmittel mit einem Bromierungsmittel oder Iodierungsmittel in Gegenwart eines Silbersalzes behandelt und die gebildeten 17α-Halogenethinyl-steroide der allgemeinen Formel III

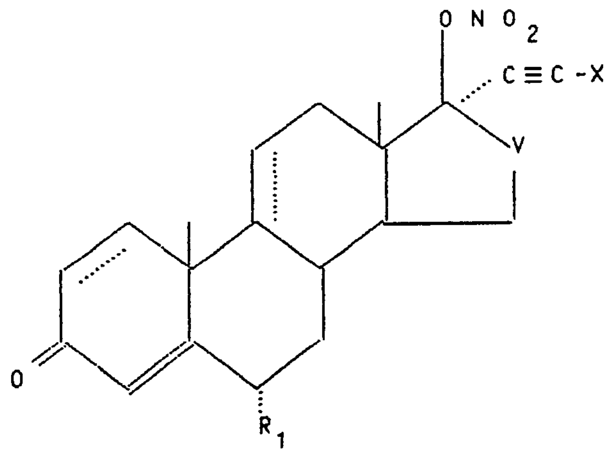

(III),

worin

...., $R_1$, V und X die oben genannte Bedeutung besitzen, in Gegenwart eines Silbersalzes mit Ameisensäure umsetzt und die gebildeten 17-Formylester der allgemeinen Formel I gegebenenfalls verseift.

Die erste Stufe des erfindungsgemäßen Verfahrens wird unter Verwendung eines Silbersalzes als Katalysator durchgeführt, der in einer Menge von 1/1000 bis 1, vorzugsweise 1/100 bis 1/10 Moläquivalenten bezogen auf das Ausgangssteroid eingesetzt wird. Geeignete Silbersalze sind beispielsweise Silbernitrat, Silberperchlorat, Silberacetat, Silbertrifluoracetat, Silberfluorid oder Silbersulfat.

Geeignete Bromierungs- bzw. Jodierungsmittel sind Bromkationen oder Jodkationen abgebende Agenzien.

Zur Bromierung eignen sich N-Bromsuccinimid, N-Brom-acetamid, N-Bromphthalimid, N-Brom-p-toluolsulfamid, N-Brom-p-toluolsulfimid, N-Bromcaprolactam, Natriumhypobromid und 1,3-Di-brom-5,5-dimethyl-hydantoin. Besonders geeignet ist N-Bromsuccinimid.

Als Jodierungsmittel dient insbesondere N-Jodsuccinimid.

Das Halogenierungsmittel wird in einer äquimolaren Menge bezogen auf das Ausgangssteroid eingesetzt, wobei jedoch ein Überschuß auch möglich ist.

Als Lösungsmittel lassen sich alle Lösungsmittel verwenden, die sich gegenüber dem Halogenierungsmittel und Silbersalz inert verhalten.

Insbesondere sind Lösungsmittel wie Ketone, z. B. Aceton, Cyclohexanon. Methylethylketon und Methylisobutylketon, cyclische Ether, z. B. Tetrahydrofuran und Dioxan, aliphatische Polyether wie Ethylenglykoldimethylether und Diethylenglykoldimethylether, aromatische Kohlenwasserstoffe wie Benzol oder Toluol, aliphatische Alkohole wie Melhanol, Ethanol und Propanol und Lösungsmittel wie Dimethylformamid, Dimethylacetamid Hexamethylphosphoortriamid und N-Methylpyrrolidon geeignet.

Die Lösungsmittel können einzeln, soweit sie das Ausgangssteroid in Lösung bringen oder suspendieren, oder auch als Mischung untereinander eingesetzt werden.

Die erfindungsgemäße Reaktion verläuft im Temperaturbereich von 0 bis 50°C und wird vorzugsweise bei Raumtemperatur durchgeführt. Sie ist in Abhängigkeit vom verwendeten Lösungsmittel innerhalb von 10 Minuten bis 20 Stunden beendet, was z. B. durch Dünnschichtchromatographie festzustellen ist.

Die zweite Stufe des erfindungsgemäßen Verfahrens kann beispielsweise unter den Bedingungen durchgeführt werden, wie sie in der DE-A-2 625 206 beschrieben sind. Überraschenderweise kann man bei dieser Reaktion anstelle von Quecksilbersalzen als Katalysator mit noch besserem Erfolg eines der oben genannten Silbersalze verwenden. Dies bringt auch insofern Vorteile, da die Verwendung von Silbersalzen zu einer geringeren Umweltbelastung führt.

Dieser Reaktionsschritt wird vorzugsweise in einem dipolarem aprotischen oder basischen Lösungsmittel durchgeführt.

Als dipolare aprotische Lösungsmittel seien beispielsweise genannt Hexamethylphosphorsäuretriamid, N-Methylpyrrolidon, Dimethylformamid, Dimethylacetamid, Dimethylsulfoxid und Sulfolan. Als basische Lösungsmittel seien beispielsweise genannt Pyridin, Toluidin und Triethylamin.

Diese Stufe des erfindungsgemäßen Verfahrens wird zweckmäßigerweise bei Raumtemperatur ausgeführt. Die Reaktion läßt sich aber auch ohne weiteres bei tieferer oder erhöhter Temperatur in einem Bereich von -10 bis 80°C durchführen.

Als Katalysator verwendet man vorzugsweise 0,01 bis 0,1 Mol Silbersalz pro Mol umzusetzendes Steroids.

Die nachfolgenden Beispiele dienen zur näheren Erläuterung des erfindungsgemäßen Verfahrens. Die angegebenen Ausbeuten beziehen sich auf Prozent der Theorie.

**Beispiel 1**

a) Zu 16,0 g 17α-Ethinyl-17β-nitrooxy-1,4-androstadien-3-on in 80 ml 1-Methyl-2-pyrrolidon werden bei Raumtemperatur 8,5 g N-Bromsuccinimid und 500 mg Silbernitrat gegeben. Nach 45 Minuten wird das Reaktionsgemisch in Eiswasser eingerührt. Das ausgefallene Produkt wird abgesaugt, in Essigester gelöst, mit Wasser gewaschen und getrocknet. Es werden 15,6 g 17α-Brom-ethinyl-17β-nitrooxy-1,4-androstadien-3-on als Schaum isoliert.

b) 13,0 g 17α-Bromethinyl-17β-nitrooxy-1,4-androstadien-3-on in 20 ml 1-Methyl-2-pyrrolidon werden bei 0°C mit 100 ml konz. Ameisensäure versetzt. Man gibt 500 mg Silbernitrat zu und läßt bei Raumtemperatur rühren. Nach 6 Stunden gibt man das Reaktionsgemisch in Eiswasser. Das ausgefallene Produkt wird abgesaugt, in Essigester gelöst, mit Wasser gewaschen und über Natriumsulfat getrocknet Es werden 9,6 g 21-Brom-17-formyloxy-1,4-pregnadien-3,20-dion erhalten.
Schmelzpunkt 191,7°C (Zers.).

c) 4,0 g 21-Brom-17-formyloxy-1,4-pregnadien-3,20-dion werden unter Argon in einem Gemisch aus 150 ml Methanol und 24 ml Wasser mit 1,3 g Kaliumhydrogencarbonat bei Raumtemperatur gerührt Nach 1 Stunde gibt man das Reaktionsgemisch in Eiswasser, saugt das ausgefallene Produkt ab, löst in Essigester, wäscht mit Wasser und trocknet über Natriumsulfat. Man erhält 3,2 g 21-Brom-17-hydroxy-1,4-pregnadien-3,20-dion.
Schmelzpunkt 189,9°C (Zers. )

**Beispiel 2**

a) Zu 5,0 g 17α-Ethinyl-17β-hydroxy-4,9(11)-androstadien-3-on [J. Org. Chem. 44, 1582 (19179)] in 50 ml Acetanhydrid tropft man bei -20°C 5,4 ml rauchende Salpetersäure. Das Reaktionsgemisch wird in Eiswasser gegeben, das ausgefallene Produkt abgesaugt, in Essigester gelöst, mit Wasser gewaschen und über Natriumsulfat getrocknet. Es werden 4,8 g 17α-Ethinyl-17β-nitrooxy-4,9(11)-androstadien-3-on erhalten. Schmelzpunkt 134,6°C (Zers.).

b) 4,0 g 17α-Ethinyl-11β-nitrooxy-4,9(11)-androstadien-3-on in 20 ml 1-Methyl-2-pyrrolidon werden bei Raumtemperatur mit 2,3 g N-Bromsuccinimid und 150 mg Silbernitrat umgesetzt. Nach 30 Minuten wird das Reaktionsgemisch in Eiswasser gegeben. Das ausgefallene Produkt wird abgesaugt, in Essigester gelöst, mit Wasser gewaschen und über Natriumsulfat getrocknet. Es werden 4,0 g 17α-Bromethinyl-17β-nitrooxy-4,9(11)-androstadien als Schaum isoliert.

c) 4,5 g 17α-Bromethinyl-17β-nitrooxy-4,9(11)-androstadien-3-on werden in 10 ml 1-Methyl-2-pyrrolidon gelöst. Bei 0°C setzt man 50 ml konz. Ameisensäure und 200 mg Silbernitrat zu. Man läßt bei Raumtemperatur rühren. Nach 8 Stunden gibt man das Reaktionsgemisch in Eiswasser. Das ausgefallene Produkt wird abgesaugt, in Essigester gelöst, mit Wasser neutral gewaschen und über Natriumsulfat getrocknet. Es verden 3,6 g 21-Brom-17-formyloxy-4,9(11)-pregnadien-3,20-dion isoliert.
Schmelzpunkt 195,3°C (Zers.).

d) 2,5 g 21-Brom-17formyloxy-4,9(11)-pregnadien-3,20-dion werden bei Raumtemperatur in 100 ml Methanol und 18 ml Wasser mit 1,3 g Kaliumhydrogencarbonat unter Argon gerührt. Nach 45 Minuten gibt man das Reaktionsgemisch in natriumchloridhaltiges Eiswasser, saugt das ausgefallene Produkt ab, wäscht mehrmals mit Wasser und trocknet im Vakuum. Es werden 2,2 g 21-Brom-17-hydroxy-4,9-(11)-pregnadien-3,20-dion [J. Org. Chem 44, 1582 (19179)] erhalten.

**Beispiel 3**

a) 2,0 g 17α-Ethinyl-17β-nitrooxy-4-androsten-3-on in 40 ml Aceton werden mit 1,1 g N-Bromsuccinimid und 100 mg Silbernitrat 90 Minuten gerüht. Das Reaktionsgemisch wird in Eis/Wasser eingerührt, das ausgefallene Produkt abgesaugt, in Essigester gelöst, mit Wasser gewaschen und getrocknet. Es werden 1,8 g 17α-Bromethinyl-17β-nitrooxy-4-androsten-3-on als Schaum erhalten.

b) 500 mg 17α-Bromethinyl-17β-nitrooxy-4-androsten-3-on werden in 1 ml 1-Methyl-2-pyrrolidon gelöst und bei 0°C mit 5 ml konz Ameisensäure versetzt. Anschließend gibt man bei Raumtemperatur 50 mg Silbernitrat zu. Nach 2 Stunden gibt man das Reaktionsgemisch in Eiswasser. Das ausgefallene Produkt wird abgesaugt, in Essigestester gelöst, mit Wasser gewaschen und über Natriumsulfat getrocknet. Es werden 380 mg 21-Brom-17-formyloxy-4-pregnen-3,20-dion erhalten. Schmelzpunkt 192,0°C (Zers.).

**Patentanspruch**

Verfahren zur Herstellung von Pregnan-Derivaten der allgemeinen Formel I

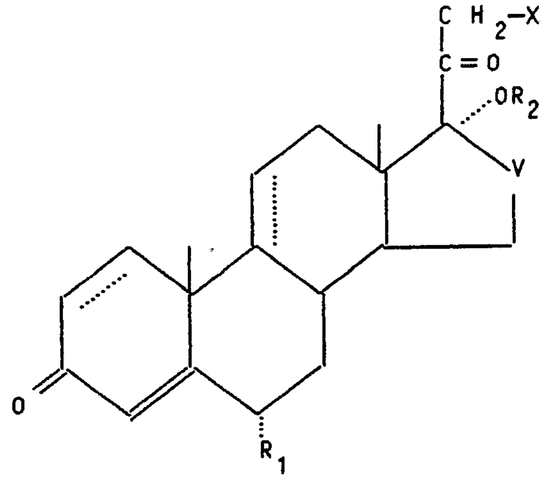

(I),

worin
.... Einfachbindungen oder Doppelbindungen darstellen,
$R_1$ ein Wasserstoffatom, ein Fluoratom oder eine Methylgruppe bedeutet,
$R_2$ ein Wasserstoffatom oder einen Formylrest symbolisiert,
X ein Bromatom oder ein Iodatom darstellt und
V eine Methylengruppe, eine Ethylengruppe, eine Ethylidengruppe oder eine Vinylidengruppe bedeutet,
dadurch gekennzeichnet, daß man ein 17-Ethinylsteroid der allgemeinen Formel II

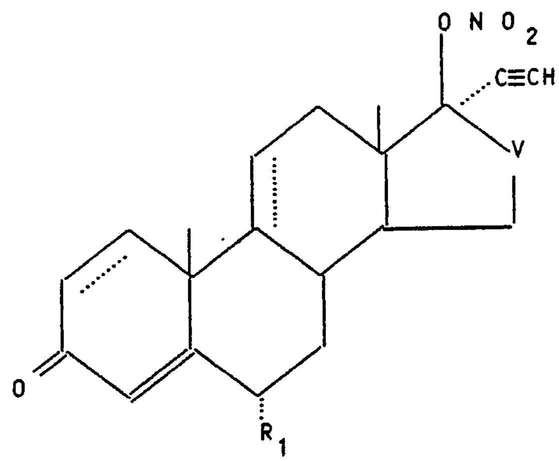

(II),

worin
...., $R_1$ und V die oben genannte Bedeutung besitzen,
dadurch gekennzeichnet, daß man die Ethinylsteroide in einem inerten Lösungsmittel mit einem Bromierungsmittel oder Iodierungsmittel in Gegenwart eines Silbersalzes behandelt und das gebildete 17-Halogensteroid der allgemeinen Formel III

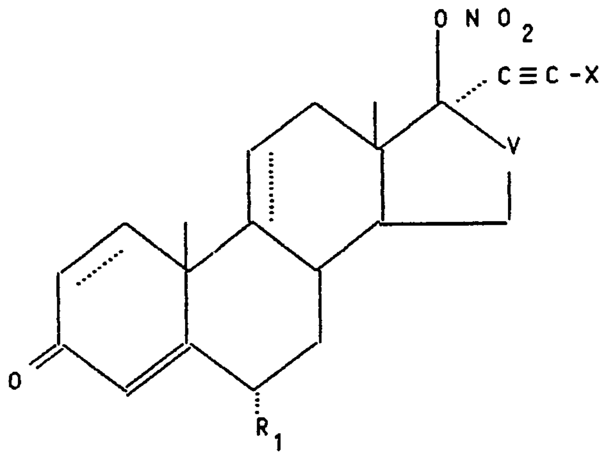

(III),

worin

.... $R_1$, V und X die oben genannte Bedeutung besitzen, in Gegenwart eines Silbersalzes mit Ameisensäure umsetzt und den gebildeten 17-Formylester der allgemeinen Formel I gegebenenfalls verseift.

## Claim

A process for the preparation of pregnane derivatives of the general formula I

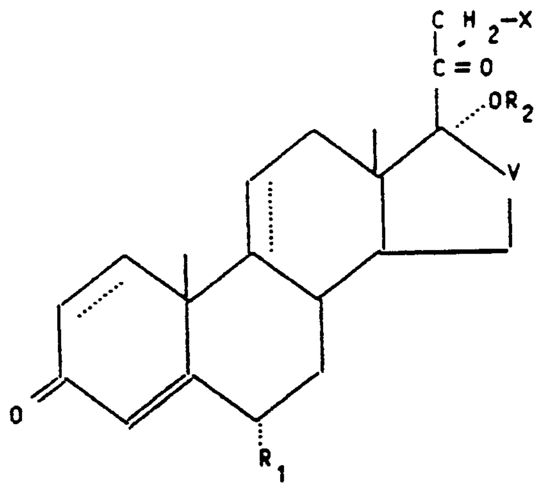

(I),

wherein

.... represent single bonds or double bonds,

$R_1$ represents a hydrogen atom, a fluorine atom or a methyl group,

$R_2$ represents a hydrogen atom or a formyl radical,

X represents a bromine atom or an iodine atom, and

V represents a methylene group, an ethylene group, an ethylidene group or a vinylidene group, characterised in that a 17-ethynyl steroid of the general formula II

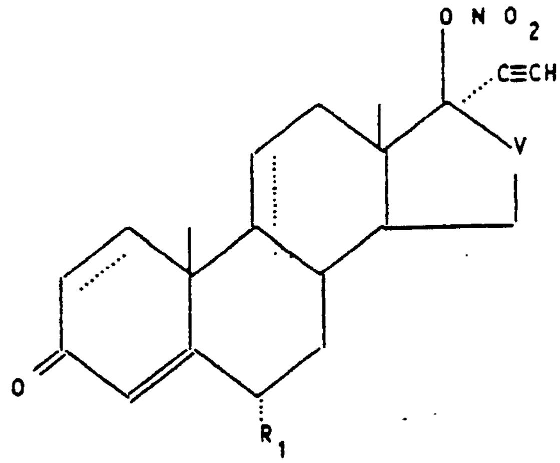

(II),

wherein

...., $R_1$ and V have the meanings given above, characterised in that the ethynyl steroids are treated in an inert solvent with a brominating agent or an iodising agent in the presence of silver salt, and the resulting 17-halo-steroid of the general formula III

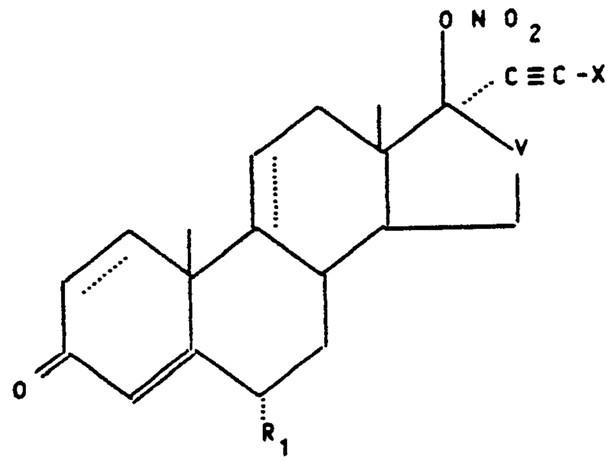

(III),

wherein

...., $R_1$, V and X have the meanings given above, is reacted in the presence of a silver salt with formic acid, and the resulting 17-formyl ester of the general formula I is optionally hydrolysed.

**Revendication**

Procédé pour préparer des dérivés du prégnane répondant à la formule générale I:

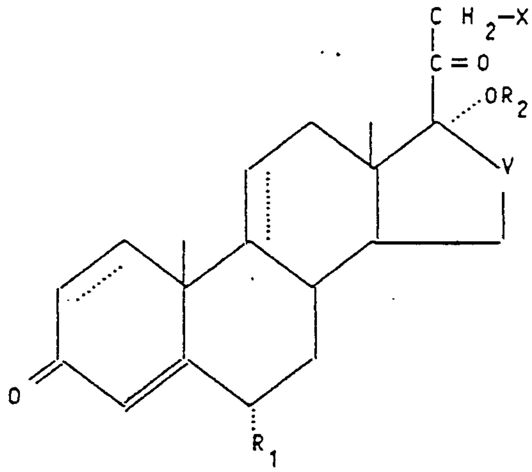

(I)

dans laquelle
les signes .... représentent chacun une liaison simple ou une liaison double,
$R_1$ représente un atome d'hydrogène, un atome de fluor ou un radical méthyle,
$R_2$ représente un atome d'hydrogène ou un radical formyle,
X représente un atome de brome ou un atome d'iode et
V représente un radical méthylène, éthylène, éthylidène ou vinylidène,
procédé caractérisé en ce qu'on traite un éthynyl-17 stéroïde répondant à la formule générale II:

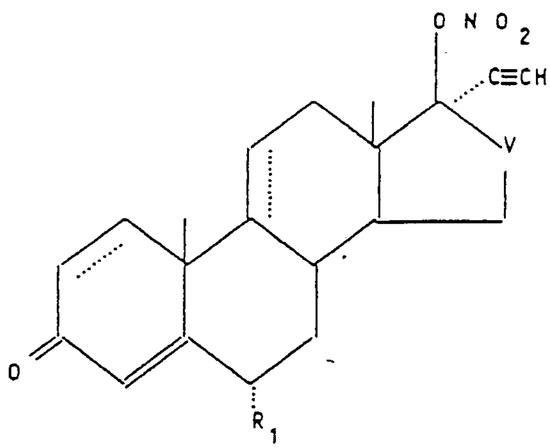

(II)

dans laquelle ...., $R_1$ et V ont les significations qui leur ont été données ci-dessus, dans un solvant inerte, par un agent de bromation ou un agent d'iodation en présence d'un sel d'argent, on fait réagir l'halogéno-17 stéroïde formé, qui répond à la formule générale III

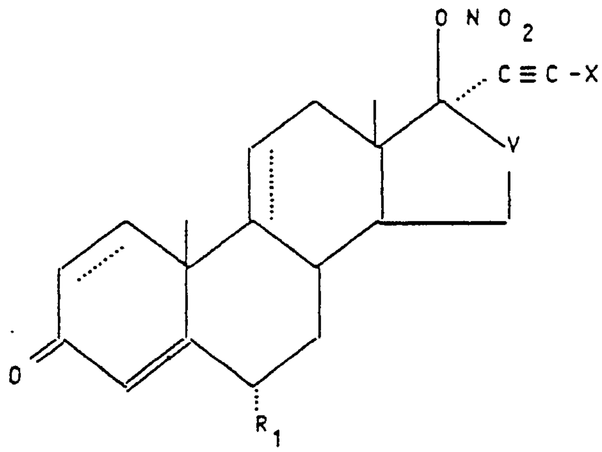

(III)

dans laquelle ...., $R_1$, V et X ont les significations qui leur ont été données ci-dessus, avec l'acide formique en présence d'un sel d'argent, et éventuellement on saponifie l'ester formique en 17 formé, qui répond à la formule générale I.